# EUROPEAN PATENT APPLICATION

(11) **EP 2 343 102 A1**
(43) Date of publication of application: **13.07.2011**
(21) Application number: 10703213.8
(22) Date of filing: 15.01.2010
(51) Int. Cl.: A61M 37/00, B29C 59/02, B81C 1/00

(54) **METHOD FOR MANUFACTURING MICRONEEDLE STAMPER**

(30) Priority: 08.10.2009 JP 2009234110
(71) Applicant: BioSerenTach Co., Ltd., Kyoto 600-8040 (JP)
(72) Inventor: HONDA Kenshin, Otsu-shi Shiga 520-0842 (JP); MOTOI Masashi, Otsu-shi Shiga 520-0842 (JP); TAKADA Kanji, Kyoto-shi Kyoto 600-8040 (JP)
(74) Representative: Haley, Stephen
(86) International application number: PCT/JP2010/050409
(87) International publication number: WO 2011/043086

(57) **Abstract**

Microneedle sheets are produced by injecting a needle raw material into a stamper formed with a concavity in a base material. However, it was not easy to increase the degree of sharpness of the concavity which corresponds to the stamper tips. A method for producing a stamper including the steps of heating a sheet-like base material and an original plate having a conical protrusion, inserting the protrusion of the original plate into the base material to form a conical concavity in the original plate, cooling the original plate with the original plate still inserted in the base material, releasing the original plate from the base material, and heating the base material.

## Description

### TECHNICAL FIELD

The present invention relates to a method for producing a female mold to produce a microneedle for injecting a drug from a skin.

### BACKGROUND ART

A microneedle sheet is a sheet in which very small needles having a length of about 1 µm to 600 µm, and a high aspect ratio of the cross-sectional diameter at the base to the length of cross-sectional diameter:length = 1:1.5 to 1:3, are arranged at a predetermined density onto a sheet substrate. The microneedle sheet is used for injecting a drug by placing it against mainly a skin portion of a human body, and inserting the microneedles (hereinafter, sometimes referred to simply as "needle") into the epidermis portion in the skin.

Since the microneedles have a length of about several hundred µm, they cause almost no pain or itchiness.
Furthermore, the microneedle portion is formed from a self-dissolving substance so that when the sheet is removed from the skin there is no harm to the body even if the microneedles remain in the skin.

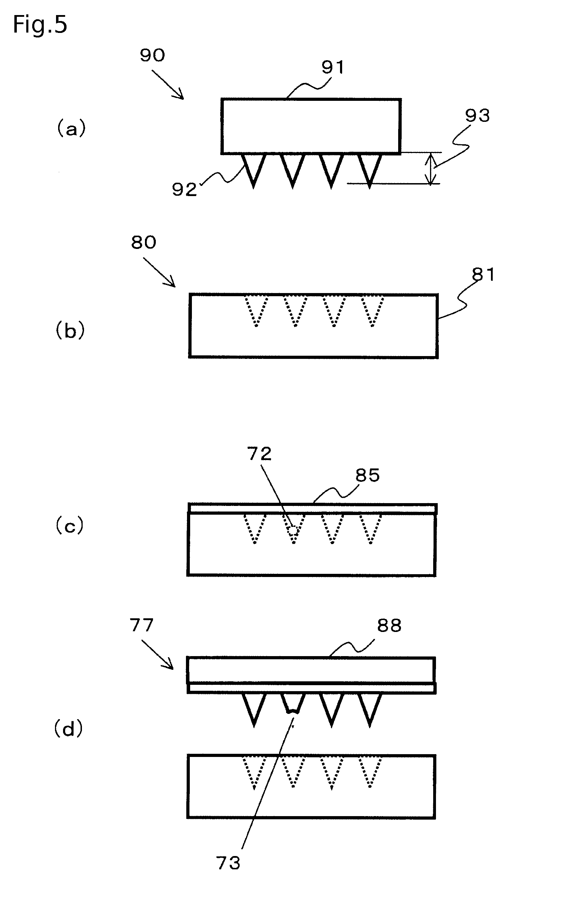

A common method for producing a microneedle sheet will now be described with reference to Fig. 5. An original plate 90 of a microneedle sheet is produced by a method such as fine machining, a vacuum treatment, or photolithography. As described above, the needle has a length 93 of several hundred µm or less, and has a conical shape. The needle cross-section may be circular, angular, elliptical or the like. Since the needle has a very small size, it is preferred to form the needle 92 by shaving from an original plate sheet 90.

Next, the original plate 90 is pressed against a stamper base material 81 to produce a stamper 80 (a mold for a microneedle sheet). Then, a resin polymer solution or a drug 85 is injected into the stamper 80. The resin polymer solution or drug 85 is dried, then stuck onto a fixing substrate 88 to be transferred thereonto and peeled off from the stamper 80, whereby a microneedle sheet 77 can be obtained (see Patent Document 1).

In the method for producing this stamper 80, rather than pressing the original plate 90 against the base material 81, the dissolved resin may be injected into the original plate 90, dried, and then peeled off (see Patent Document 2).

### LIST OF PRIOR ART DOCUMENTS

### PATENT DOCUMENTS

[Patent Document 1] Japanese Patent Application Laid-Open No. 2008-245955
[Patent Document 2] Japanese Patent Application Laid-Open No. 2008-006178
[Patent Document 3] Japanese Patent Application Laid-Open No. 2009-083125

### SUMMARY OF THE INVENTION

### PROBLEMS TO BE SOLVED BY THE INVENTION

For a microneedle to penetrate the skin, the tip of the microneedle has to have a degree of sharpness which is as high as possible. Furthermore, a microneedle sheet is produced by injecting a needle raw material into a produced stamper. Therefore, the bottom of a concavity of the stamper into which the raw material is injected (corresponding to the tip of the microneedle) has to be a hollow with a high degree of sharpness. However, there has been the problem that it is not easy to form a hollow having a high degree of sharpness in the base material.

Furthermore, from the perspective of injecting the needle raw material into the stamper, since the concavities in the stamper have extremely small dimensions, there has been the problem that the raw material of the microneedles is not easily filled into the concavities of the stamper due to air and surface tension of the material.

More specifically, in Fig. 5(c), when a resin polymer solution or a drug 85 is injected into the stamper 81, air 72 is present in a concavity of the stamper. When peeling from the stamper, at a portion (73) where the air 72 was present, the needle can only be formed in an incomplete shape (Fig. 5(d)).

This problem is also described in Patent Documents 2 and 3. Patent Document 2 describes carrying out the filling of the raw material into the stamper under a reduced pressure. Patent Document 3 describes filling a raw material into a stamper concavity while applying a pressure from the raw material side. However, when providing many microneedles, there is the problem that the raw material is not filled in some of the concavities, which means that not all of the planned microneedles can be formed.

### MEANS FOR SOLVING THE PROBLEMS

The present invention was created in consideration of the above-described problems. Specifically, the present invention provides a method for producing a stamper comprising the steps of:
heating a sheet-like base material and an original plate having a conical protrusion;
forming a conical concavity by inserting the protrusion of the original plate into the base material;
cooling the original plate with the original plate still inserted in the base material;
releasing the original plate from the base material; and
heating the base material.

Furthermore, the method for producing a stamper according to the present invention is characterized in that, in the step of forming a conical concavity, the protrusion of the original plate is inserted into the base material until the protrusion penetrates the base material.

### ADVANTAGES OF THE INVENTION

In the present invention, a conical concavity is provided by inserting an original plate having a conical protrusion into a warmed stamper member. The original plate is cooled in that state, and then released from the stamper member. Therefore, the concavity is formed with stress still remaining in the stamper member which was deformed by the conical protrusion. If the stamper member is heated after releasing the conical protrusion, the stress is released, and re-deformation occurs so that the concavity is filled in. Consequently, the base material is re-deformed in a direction in which the bottom of the concavity is narrowed, and this enables a concavity with a high degree of sharpness to be formed.

Furthermore, if a through-hole which penetrates as far as an opposite face of the stamper is formed in the bottom of the concavity in advance, and the stamper member is re-deformed by the above-described method, a very small through-hole can be formed. The concavities of the stamper having very small through-holes allow air to easily escape even if air accumulates during the coating of the microneedle member. Consequently, a microneedle sheet can be obtained on which all of the planned microneedles are formed by enabling a raw material to be filled into all of the concavities of the stamper.

### BRIEF DESCRIPTION OF THE DRAWINGS

Fig. 1 is a schematic diagram of a stamper according to the present invention.
Fig. 2 is a diagram illustrating a production process of the stamper according to the present invention.
Fig. 3 is a diagram illustrating the production process of the stamper according to the present invention in detail.
Fig. 4 is a diagram illustrating a production process of a microneedle sheet using the stamper according to the present invention.
Fig. 5 is a diagram illustrating a production process of a microneedle sheet using a conventional stamper.

### BEST MODE FOR CARRYING OUT THE INVENTION

### (Embodiment 1)

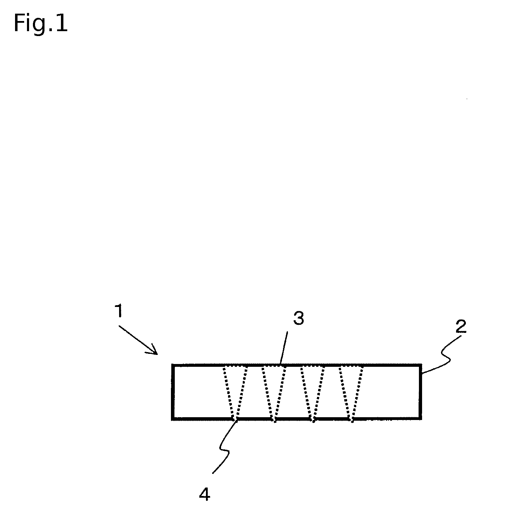

Fig. 1 illustrates a stamper 1 of a microneedle sheet according to the present invention. The stamper 1 is formed with conical concavities 3 in a sheet-like base material 2. The material of the base material 2 is not especially limited. However, considering that such a material will be used together with a medicine, a material which does not easily contaminate or does not have an effect on the human body is preferred. Furthermore, the stamper 1 according to the present invention forms a concavity shape having a tip with a high degree of sharpness by thermal shrinkage. Therefore, it is preferred to use a plastic such as PTFE, polypropylene, and polyethylene.

A through-hole 4 which penetrates to another face of the base material is formed in each of bottom portions of the concavities 3. The through-hole 4 preferably has a small diameter, because the through-hole 4 will act as the portion which forms the tip of the microneedle. Functionally, the through-hole 4 does not have to be a hole with a finite diameter which is constantly open, so long as air which has accumulated in the concavity can escape in order for the raw material to be filled into the concavities 3. Specifically, as long as air escapes when a pressure is applied, the through-hole may normally look like it is closed. More specifically, the through-hole preferably has a diameter of several tens of microns or less.

The microneedle arrangement density and arrangement distribution are not especially limited, and these may be determined as necessary based on the application and purpose. In the drawings, for simplicity, a stamper for producing a microneedle sheet having four microneedles is illustrated.

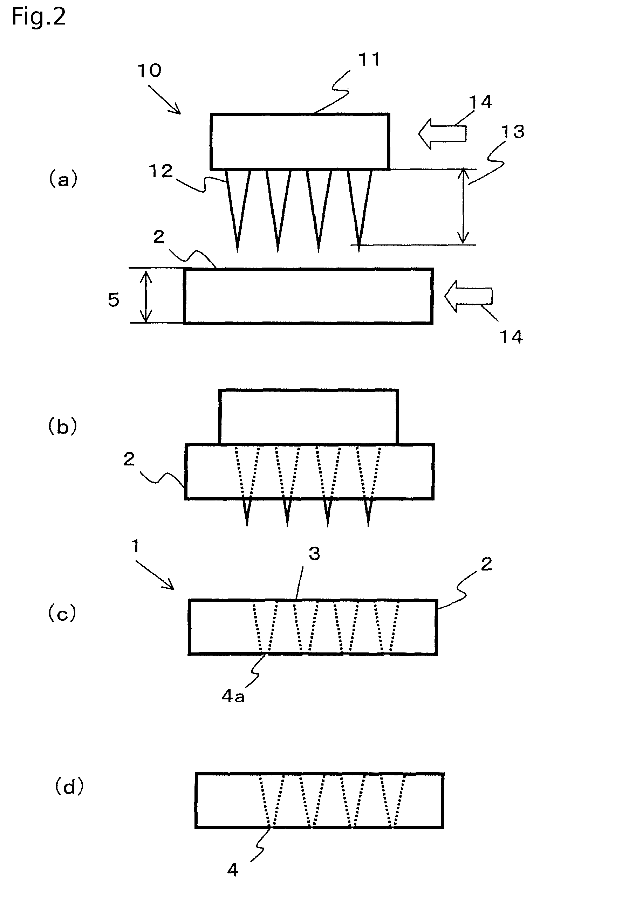

Next, examples of the method for producing the stamper according to the present invention will be described. With reference to Fig. 2(a), an original plate 10 includes a flat body 11 on which conical protrusions 12 are formed. The material for the original plate 10 is not especially limited, but it is preferred to use a metal which has excellent workability. Furthermore, in the present invention, the base material is heated to a temperature near its softening point. Therefore, a material capable of maintaining hardness at around that temperature is preferred. Since the conical protrusions are very small, a material which can be easily shaved is preferred. Examples of the material used include copper, aluminum, nickel, silicon and the like. In addition to the processing of the protrusions 12 by shaving the protrusions from the body 11, the protrusions 12 may also be formed by a method using photolithography.

The protrusions 12 stand on the body 11 with a height 13 of from about 1 µm to 600 µm. The reason for the height setting is as follows. Although the epidermis into which the drug or the like is injected using the microneedle sheet is formed from the stratum corneum, stratum granulosum, stratum spinosum, and stratum basale, the thickness of the stratum corneum is different depending on the area of the body. Thus, the height of the microneedles can change depending on the area of the body into which transdermal administration is to be carried out and the drug.

The protrusions 12 have a needle shape with a comparatively high aspect ratio of the cross-sectional diameter at the base of the microneedle formed by the stamper to the length of cross-sectional diameter:length = 1:1.5 to 1:3. This is because the microneedles may change depending on the area to be used. The cross-sectional shape is not especially limited. Examples of shapes which may be preferably used include circular, elliptical, rectangular including square, and the like. Furthermore, a groove may be formed on the surface of the protrusions. This is because a microneedle which reflects the shape of such a groove can be smoothly inserted into the skin.

A thickness 5 of the base material 2 is thinner than a length 13 of the protrusions 12. The tip of the protrusions 12 is for penetrating the base material 2 to form the through-hole 4. The diameter of the through-hole 4 formed in the base material 2 can be set based on the tip shape of the protrusions 12 and the thickness 5 of the base material 2 so as to be about several tens of microns. This is because, in the present invention, the through-hole 4 may be shrunk by thermal shrinkage, which means that a through-diameter 4a formed when a protrusion 12 of the original plate 10 penetrates the base material 2 may be several times larger than the through-hole 4 of the stamper 1.

First, the original plate 10 and the base material 2 are subjected to pre-heating 14. The heating temperature is a temperature lower than the melting point of the base material 2. The heating temperature is preferably near the softening point temperature of the base material 2. This is because in the formation of the concavities of the stamper according to the present invention, a step of causing plastic deformation with stress remaining in the base material 2 is carried out. If the temperature of the original plate 10 is higher than the melting point of the base material 2, the base material 2 melts, so that the concavities 3 cannot be formed in the shape of the protrusions 12 of the original plate 10. Furthermore, if the temperature of the original plate 10 is too low compared with the temperature of the base material 2, the stress which is required during the re-deformation does not remain.

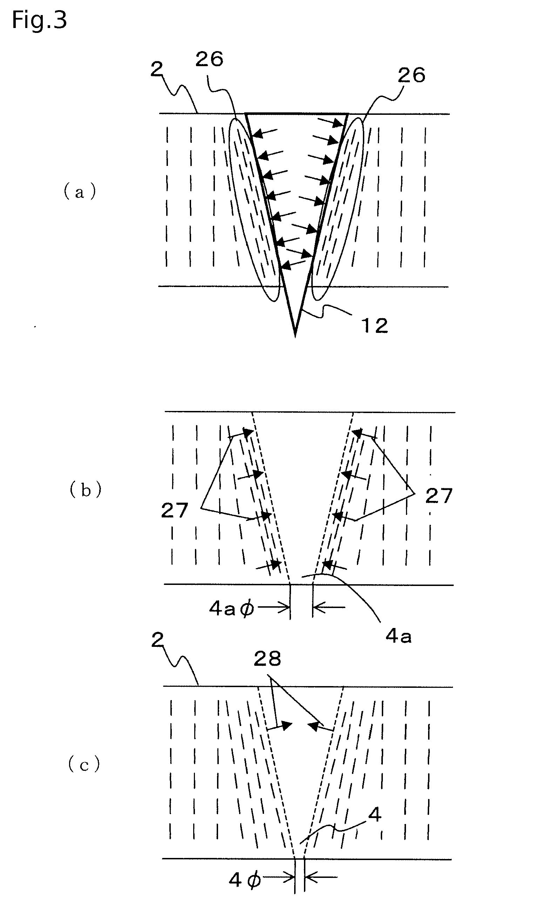

Next, the heated original plate 10 is thrust into the heated base material 2 (Fig. 2(b)). Then, with the original plate 10 still stuck in the base material 2, the original plate 10 is cooled to room temperature, and then released from the base material (Fig. 2(c)). Fig. 3(a) illustrates an enlarged view of a portion where the protrusion 12 of the heated original plate 10 was thrust into the heated base material 2. At the potion where the protrusion 12 is inserted into the base material 2 (reference numeral 26), the base material is pushed wider by the protrusion 12. Consequently, the molecules are in a compressed state. This compressed portion is referred to as a "concavity surface layer 26".

With reference to Fig. 3(b), when the cooled original plate 10 is released, the shape is maintained in a state in which the stress 27 is still accumulated in the concavity surface layer 26 having the through-hole. In Fig. 3(b), while a reference numeral was given for the two arrows on the left and right, the other arrows also represent stress. Further, the through-hole which is formed at this stage is the through-hole 4a.

Returning to Fig. 2(d), after the cooling, the base material 2 from which the original plate 10 was released is re-heated. The heating temperature at this stage is near the softening point temperature thereof, and preferably at or lower than the melting point thereof. This is because if the heating temperature is in this range, deformation can occur due to the release of stress in the concavity surface layer.

With reference to Fig. 3(c), since the stress is released at the concavity surface layer of the heated base material 2, the surface layer moves toward (28) a free space. As a result of the re-deformation due to this thermal shrinkage, the through-hole shrinks, whereby a concavity having a high degree of sharpness can be formed. Furthermore, compared with a case when the original plate of Fig. 3(b) was released, a through-hole diameter (4aϕ) decreases (4ϕ) due to the shrinkage. More specifically, for an example of a polyethylene base material 2, if the base material 2 is held at 90°C for about 10 seconds, the diameter of the through-hole can be shrunk from 40 µm to 10 µm, so that a very small microneedle can be produced.

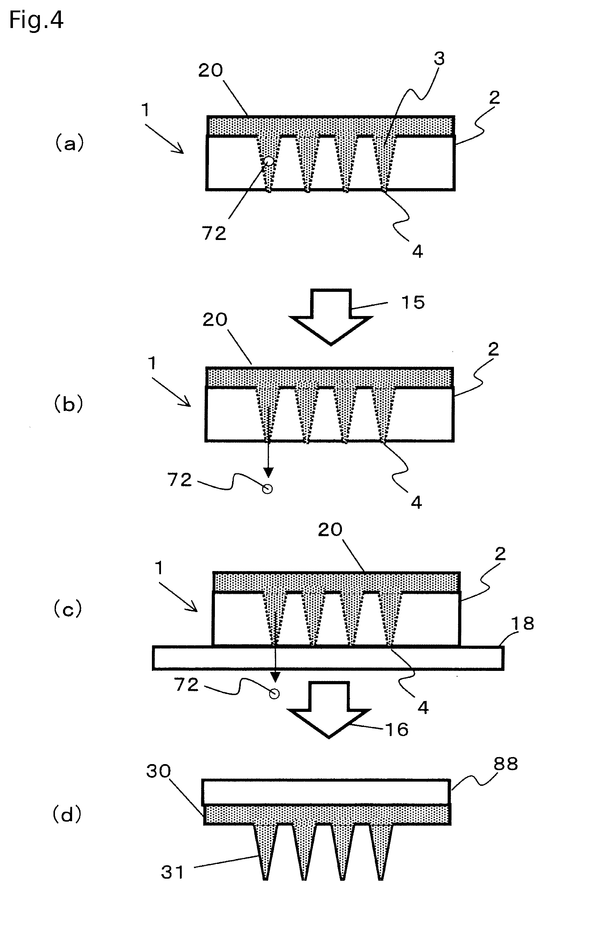

The microneedle sheet is produced using the thus-produced stamper. With reference to Fig. 4, the microneedle sheet is produced by coating a needle raw material 20 on the stamper 1 (Fig. 4(a)). As the needle raw material 20, it is preferred to use a material which is discharged without remaining in the body. The reason for this is as follows. When the microneedle sheet is peeled off from the skin, the needle portions may break or be pulled out of the sheet, so that some needles cannot be recovered. Therefore, in such cases, it is safer if the material is absorbed by the body. Specifically, the needle raw material is preferably a material which has a thread-forming substance and the like as a main component. It is also preferred to pre-mix the drug in the needle raw material. Here, the term "drug" refers to a pure chemical substance having a physiologically active effect. Examples of such a substance include peptide/protein drugs such as insulin, growth hormone, erythropoietin, and interferon, polymer drugs, vitamin C and the like. The drug may also be a substance having any one of them as a main component.

As illustrated in Fig. 4(a), the stamper 1 according to the present invention has the through-holes 4 formed in the bottoms of concavities 3. Therefore, to inject the needle raw material into the stamper, the needle raw material is filled into the concavities just by coating. Specifically, even if air 72 is present at the bottoms of the concavities 3 of the stamper 1, the air can easily be discharged via the through-holes 4, so that the needle raw material is filled into the concavities 3. Obviously, a pressure 15 may be applied after the coating (Fig. 4(b)). If a pressure is applied, air which has accumulated at the bottoms of the concavities 3 of the stamper can easily escape.

Furthermore, the stamper 1 may be arranged on a flat mount 18 formed by a porous solid, and the needle raw material 20 can be coated while applying a negative pressure 16 from the back face of the flat mount 18 (see Fig. 4(b)). In this case too, the needle raw material 20 can be easily injected into the concavities 3 of the stamper. Moreover, it is more preferred to apply a pressure 15 from the top face of the flat mount 18 and apply the negative pressure 16 from the back face.

After the needle raw material 20 is coated on the stamper 1, the needle raw material 20 is dried and peeled from the stamper, thereby completing a microneedle sheet 30 (see Fig. 4(3)). The peeling from the stamper 1 is carried out by sticking the fixing substrate 88 from the back face of the dried needle raw material 20, and peeling from the stamper. If a drug was not added into the microneedle sheet 30 peeled from the stamper, after being peeled from the stamper, the drug is dispersed or the like in a needle portion 31.

Furthermore, the thus-produced microneedles 31 are formed in a conical shape having a comparatively high aspect ratio in which the ratio of the cross-sectional diameter at the base of the microneedle 31 to the length thereof is cross-sectional diameter:length = 1:1.5 to 1:3.

Thus, in the stamper according to the present invention, a very small through-hole which penetrates as far as an opposite face of the stamper is drilled into the bottom of a concavity forming a needle. Therefore, even if air is in the concavity, the air can easily escape, which allows a microneedle sheet that can produce a needle having a high degree of sharpness to be obtained.

### INDUSTRIALAPPLICABILITY

The present invention can not only be used for a microneedle sheet which injects a drug into the epidermis, but can also be widely used as a method for generating very small protrusions on a substrate.

### DESCRIPTION OF REFERENCE NUMERALS

- 1: stamper
- 2: base material
- 3: concavity
- 4: through-hole
- 5: thickness of base material
- 10: original plate
- 11: body of original plate
- 12: protrusion
- 13: height of protrusion
- 14: heating
- 15: pressure
- 16: negative pressure
- 18: flat mount made of porous solid
- 20: needle raw material
- 26: concavity surface layer
- 27: stress
- 28: moving direction of concavity surface layer
- 30: microneedle sheet
- 31: needle
- 72: air

- 77: microneedle sheet
- 80: conventional stamper
- 81: conventional stamper base material
- 85: needle raw material
- 88: fixing substrate
- 90: original plate
- 91: body
- 92: protrusion
- 93: length of protrusion

## Claims

1. A method for producing a stamper comprising the steps of:
heating a sheet-like base material and an original plate having a conical protrusion;
forming a conical concavity by inserting the protrusion of the original plate into the base material;
cooling the original plate with the original plate still inserted in the base material;
releasing the original plate from the base material; and
heating the base material.

2. The method for producing a stamper according to claim 1, wherein, in the step of forming a conical concavity, the protrusion of the original plate is inserted into the base material until the protrusion penetrates the base material.
